(19) Europäisches Patentamt
European Patent Office
Office européen des brevets

(11) Numéro de publication: **0 349 354**
**A1**

(12) **DEMANDE DE BREVET EUROPEEN**

(21) Numéro de dépôt: **89400905.9**

(22) Date de dépôt: **31.03.89**

(51) Int. Cl.⁵: **C 07 K 7/00**
G 01 N 33/569, C 07 K 1/00,
C 07 K 15/00

(30) Priorité: **01.04.88 FR 8804405**
**01.04.88 FR 8804406**

(43) Date de publication de la demande:
**03.01.90 Bulletin 90/01**

(84) Etats contractants désignés:
**AT BE CH DE ES FR GB GR IT LI LU NL SE**

(71) Demandeur: **INSTITUT PASTEUR**
**25-28, rue du Docteur Roux**
**F-75724 Paris Cédex 15 (FR)**

(84) Etats contractants désignés:
**AT BE CH DE ES FR GB GR IT LI LU NL SE**

(71) Demandeur: **CENTRE NATIONAL DE LA RECHERCHE SCIENTIFIQUE**
**15, quai Anatole France**
**F-75007 Paris (FR)**

(84) Etats contractants désignés: **FR**

(72) Inventeur: **Montagnier, Luc**
**21 rue de Malabry**
**92350 Le Plessis Robinson (FR)**

**Rochat, Hervé**
**Ch. des Saugeonnes**
**13120 Mimet (FR)**

**Bahraoui, El Mustapha**
**14 rue du Cdt Marchand**
**94230 Cachan (FR)**

**Chamaret, Solange**
**324 rue Lecourbe**
**75015 Paris (FR)**

**Ferris, Stéphane**
**90 rue Cambronne**
**75015 Paris (FR)**

**Granier, Claude**
**23 Hameau Saint-Félix**
**13800 Istres (FR)**

**Van Rietschoten Jurphaar**
**Val de la Torse, AZ**
**13100 Aix en Provence (FR)**

**Sabatier Jean-Marc**
**58 av. de la Timone**
**13010 Marseille (FR)**

(74) Mandataire: **Gutmann, Ernest et al**
**S.C. Ernest Gutmann - Yves Plasseraud 67, boulevard Haussmann**
**F-75008 Paris (FR)**

(54) **Peptides PF10 à PF19 d'un rétrovirus HIV, procédé de synthèse de ces peptides, leur utilisation notamment pour le diagnostic.**

(57) L'invention concerne des peptides capables de former un complexe immunologique avec des anticorps anti-protéine F d'un rétrovirus susceptible de provoquer un SLA ou un SIDA, caractérisés en ce qu'ils comportent au plus 70 aminoacides et possèdent une séquence d'aminoacides correspondant à une partie de la protéine F d'un HIV ou à une variante de cette séquence néanmoins telle qu'elle soit immunologiquement reconnue par des anticorps anti-protéines F de rétrovirus HIV.

EP 0 349 354 A1

## Description

# PEPTIDES PF10 à PF19 D'UN RETROVIRUS HIV - PROCEDE DE SYNTHESE DE CES PEPTIDES- LEUR UTILISATION NOTAMMENT POUR LE DIAGNOSTIC

L'invention concerne des peptides ayant des propriétés immunologiques, le cas échéant immunogènes, en commun avec la protéine F d'un rétrovirus capable de provoquer chez l'homme des syndrômes de lymphadénopathies (SLA) susceptibles d'être relayés par un syndrôme d'immunodéficience acquise (SIDA).

Jusqu'à présent, plusieurs rétrovirus ayant une responsabilité dans le développement d'un SLA ou d'un SIDA, ont été isolés et caractérisés. Ainsi un premier virus dénommé LAV-1 ou HIV-1 a été isolé et décrit dans la demande de brevet GB.83/24.800 et une demande EP.84/401.834 du 14/09/84. Ce virus a également été décrit par F.Barre Sinoussi et al. dans Science, 220 n° 45-99, 20, pages 868-871.

Des variants de ce virus HIV-1 désignés par LAV ELI et LAV MAL, ont également été isolés, caractérisisés et décrits dans la demande de brevet EP. 84/401.8301.834.

L'isolement et la caractérisation de rétrovirus appartenant à une classe distincte et n'ayant qu'une parenté immunologique réduite avec les précédents, ont été décrits dans la demande de brevet européen n° 87/400.151.4 publiée sous le n° 239.425. Ces rétrovirus qui ont été regroupés sous la désignation HIV-2, ont été isolés chez plusieurs malades africains présentant des symptômes d'une lymphadénopathie ou d'un SIDA.

Les auteurs de la demande de brevet européen publiée sous le n° 0226513 se sont intéressés à la recherche des peptides présents dans une protéine codée par le génome d'un virus HIV, et possédant des séquences peptidiques communes avec des antigènes MHC (abréviation anglaise de "self major histocompatibility complexes"). De telles séquences peptidiques non spécifiques des rétrovirus HIV, ont selon la demande de brevet EP.0226513 la capacité d'inhiber l'interaction du virus responsable du SIDA avec les récepteurs T4 portés par les lymphocytes T4 et plus généralement d'exercer un effet immunomodulateur à l'égard respectivement des interactions entre les macrophages et les lymphocytes T auxiliaires, d'une part, et des interactions entre ces lymphocytes T auxiliaires et les lymphocytes B, d'autre part, et ce lorsque ces interactions sont normalement médiées par des récepteurs lymphocytaires codés par des complexes majeurs d'autohistocompatibilité de classe II (antigènes HLA de classe II chez l'homme).

L'étude de ces rétrovirus HIV-1 et HIV-2 a conduit à l'obtention de leurs séquences d'ADN complémentaires puis à la caractérisation des séquences nucléotidiques spécifiques codant pour d'autres protéines que celles décrites précédemment, notamment pour la protéine F de HIV-1 décrite dans la publication Bruno Guy et al. Nature 1987, vol. 33, p. 266 à 269.

On connaissait les propriétés antigéniques de la protéine F vis-à-vis d'anticorps contenus dans un sérum de patient infecté par un rétrovirus capable de provoquer un SLA ou un SIDA et plus particulièrement vis-à-vis d'anticorps dirigés contre cette protéine F.

Les inventeurs ont constaté que certaines séquences peptidiques sélectionnées à partir de la séquence connue de la protéine F, pour différents isolats de rétrovirus HIV-1 ou HIV-2, présentent un intérêt particulier pour la détection d'une infection par l'un des rétrovirus décrits plus haut.

Ces séquences peptidiques, spécifiques des rétrovirus HIV appartiennent à une classe de protéines de régulation et par opposition aux protéines virales de structure, elles sont excrétées par la cellule infectée.

De façon tout à fait intéressante les inventeurs ont remarqué que certains peptides de la protéine F (encore désignés par PF) sont reconnus par des anticorps induits dans un hôte infecté par un rétrovirus du type HIV à un stade très précoce de l'infection par le virus.

Cette observation démontre s'il en est besoin l'intérêt que peuvent représenter de tels peptides pour la détection à un stade précoce d'une infection chez l'homme par un rétrovirus des classes HIV-1 ou HIV-2.

L'invention concerne à cet égard des peptides de la protéine F d'un rétrovirus capable de provoquer un SLA ou un SIDA, qui présentent des propriétés immunologiques, en commun avec celles de cette protéine F et qui ont la capacité d'être reconnus par des anticorps contre cette protéine, présents dans un échantillon biologique, notamment un tissu ou un liquide biologique, d'un hôte infecté par un rétrovirus HIV notamment de la classe HIV-1 ou de la classe HIV-2.

L'invention vise en outre des peptides de la protéine F d'un rétrovirus HIV-1 ou HIV-2, ayant des propriétés immunogènes, ou susceptibles d'être rendus immunogènes in vivo.

Les peptides de l'invention peuvent être obtenus par isolement à partir de la séquence complète de la protéine F d'un rétrovirus déterminé ou encore par synthèse chimique.

La figure 1 représente la séquenoe peptidique de la protéine F pour deux isolats, correspondant l'un au rétrovirus HIV-2, l'autre au rétrovirus HIV-1. Les correspondances entre les acides aminés et leur code à une lettre sont les suivantes :

M Méthionine
L Leucine
I Isoleucine
V Valine
F Phénylalanine
S Sérine
P Proline
T Thréonine

A Alanine
Y Tyrosine
H Histidine
Q Glutamine
N Asparagine
K Lysine
D Acide Aspartique
E Acide glutamique
C Cystéine
W Tryptophane
R Arginine
G Glycine

Les peptides de l'invention, obtenus éventuellement après modification de l'enchaînement d'acides aminés, à partir d'un isolat d'un rétrovirus ou encore obtenus par synthèse sont tels qu'ils présentent une parenté de séquence ou (homologie) avec la séquence peptidique correspondante de la protéine F d'un rétrovirus de la classe HIV-1 ou/et d'un rétrovirus de la classe HIV-2, suffisante pour que les propriétés immunologiques de cette séquence vis-à-vis des anticorps formés dans un milieu biologique (notamment un sérum) d'un hôte infecté par l'un ou l'autre des virus HIV-1 ou HIV-2 soient conservées.

La modification de l'enchaînement d'acides aminés dont il est question ci-dessus, peut consister dans l'addition à cet enchaînement correspondant à la séquence d'acides aminés de la protéine F du rétrovirus HIV, à partir de laquelle est déterminé le peptide, un acide aminé de type cystéine extrinsèque, en position N- ou C-terminale, afin de pouvoir coupler de façon univoque (dirigée) le peptide sur une protéine transporteuse par exemple.

A cet égard, les peptides selon l'invention, capables de former un complexe immunologique avec des anticorps anti-protéine F d'un rétrovirus susceptible de provoquer un SLA ou un SIDA, sont caractérisés en ce qu'ils comportent au plus 70 aminoacides formant une séquence correspondant à une partie de la protéine F d'un rétrovirus HIV ou à une variante de cette séquence néanmoins telle qu'elle soit immunologiquement reconnue par des anticorps anti-protéine F de rétrovirus HIV.

Dans un autre mode de réalisation de l'invention, le peptide est sélectionné en fonction de sa capacité à être reconnu par des anticorps anti-protéine F d'un hôte infecté spécifiquement par l'un ou l'autre des rétrovirus HIV-1 ou HIV-2.

Un premier peptide PF16 (HIV-1) conforme à l'invention est caractérisé par tout ou partie de la formule :
XGM-D-E--VL-W-F-S-LA--H-ARE-HPE--K-CZ
dans laquelle :
- les tirets "-" figurent des acides aminés variables choisis de manière à ce que le peptide final conserve sa capacité à être reconnu par des anticorps formés lors d'une infection par l'un des rétrovirus précédents,
- les groupes X représentent soit un groupe $NH_2$ libre ou amidé, notamment par un ou deux groupes alcoyle comprenant de 1 à 5 atomes de carbone, soit un groupe peptidique comprenant de 1 à 5 aminoacides, dont l'amino-acide N-terminal présente lui-même un groupe $NH_2$ libre ou amidé comme précédemment indiqué, et les groupes Z représentent, soit un groupe-OH libre ou alcoxyle et contenant alors un groupe alcoyle comprenant de 1 à 5 atomes de carbone, soit un groupe peptidique comprenant de 1 à 5 aminoacides, dont l'aminoacide C- terminal présente lui-même un groupe-OH libre ou alcoxyle, comme précédemment indiqué, les groupes de 1 à 5 acides aminés le cas échéant contenus dans X ou Z ou dans les deux à la fois ainsi que les acides aminés remplaçant les tirets étant tels, que leur présence n'est pas incompatible avec les propriétés immunologiques, le cas échéant immunogènes, des peptides formés, notamment leur capacité à être reconnus par des sérums humains contenant des anticorps contre la protéine F d'un rétrovirus HIV-1 et plus particulièrement des anticorps monospécifiques contre le peptide ci-dessus.

Des peptides préférés répondant à la structure précédente sont les suivants:
XGMDDPEREVLEWRFDSRLAFHHVARELHPEYFKNCZ
XGMEDAEKEVLVWRFDSKLAFHHMARELHPEYYKDCZ
XGMEDAEREVLKWKFDSSLALRHRAREQHPEYYKDCZ
XGMEDPERQVLKWRFNSRLAFEHKAREMHPEFYKNZ

Des peptides particulièrement préférés sont donnés par les enchaînements d'acides aminés suivants:
GMDDPEREVLEWRFDSRLAFHHVARELHPEYFKNC
GMEDAEKEVLVWRFDSKLAFHHMARELHPEYYKDC
GMEDAEREVLKWKFDSSLALRHRAREQHPEYYKDC
GMEDPERQVLKWRFNSRLAFEHKAREMHPEFYKN

D'autres peptides appartenant à la protéine F couverts par l'invention caractérisés par leur capacité à former un complexe immunologique avec des anticorps dirigés contre la protéine F contenus dans un sérum humain infecté par un rétrovirus HIV-1 sont les peptides suivants :
PF11:XGGKWSKSSVVGWPTVRERMRRAEPAADGVGAYCZ
PF12:XGGKWSKSSVVGWPTVRERMRRAEPAADGVGAASRDLEKHGAITSSNTA ATNAACAWLEAQEEEEVGZ
PF13:XASRDLEKHGAITSSNTAATNAACAWLEAQEEEEZ
PF14:XCVGFPVTPQVPLRPMTYKAAVDLSHFLKEKGGLEGLIHSQRRQDILZ

PF15:XCGYFPDWQNYTPGPGVRYPLTFGWCYKLVPVEPDKVEEANKGENTSLL HPVZ
PF17:XCYKLVPVEPDKVEEANKGENTSLLHPVSLHGMDDPEREVLEWRFDSRL AFHHVARELHPEYFKNCZ
PF18:XCKGGLEGLIHSQRRQDILDLWIYHTQGYFPDZ

L'invention vise aussi des peptides ayant les mêmes séquences d'acides aminés que les précédentes mais comportant, au niveau de certains radicaux cystéine des groupements protecteurs acétamino-méthyle (acm).

PF11:XGGKWSKSSVVGWPTVRERMRRAEPAADGVGAYC(acm)Z
PF12:XGGKWSKSSVVGWPTVRERMRRAEPAADGVGAASRDLEKHGAITSSNTA ATNAAC(acm)AWLEAQEEEEVGZ
PF13:XASRDLEKHGAITSSNTAATNAAC(acm)AWLEAQEEEEZ
PF14:XC(acm)VGFPVTPQVPLRPMTYKAAVDLSHFLKEKGGLEGLIHSQRRQ DILZ
PF15:XCGYFPDWQNYTPGPGVRYPLTFGWC(acm)YKLVPVEPDKVEEANKGE NTSLLHPVZ
PF17:XC(acm)YKLVPVEPDKVEEANKGENISLLHPVSLHGMDDPEREVLEWR FDSRLAFHHVARELHPEYFKNC(acm)Z
PF18:XC(acm)KGGLEGLIHSQRRQDILDLWIYHTQGYFPDZ

L'invention englobe encore des variants de ces peptides, dans la mesure où ils conservent leurs propriétés immunologiques vis-à-vis des anticorps dirigés contre la protéine F, présents dans un sérum humain infecté par un rétrovirus HIV-1. Des variants de ces peptides sont plus particulièrement les peptides obtenus en mettant les peptides ci-dessus en alignement avec ceux des peptides qui leur correspondent pour les autres isolats de HIV-1 décrits dans la figure 3.

Cette figure 3 représente les alignements des séquences de la protéine F pour 4 isolats du virus du SIDA. L'isolat $LAV_{BRU}$ est pris comme référence et les seules différences de $ARV_2$, $LAV_{MAL}$ et $LAV_{ELI}$ avec $LAV_{BRU}$ sont notées.

L'invention vise aussi les peptides ci-dessus dépourvus des acides aminés de type cystéine extrinsèque dont il a été question ci-dessus, et qui ont pu être ajoutés lors de la synthèse chimique pour les besoins du couplage.

Les tirets correspondent à des délétions introduites dans les alignements.

D'autres peptides conformes à l'invention sont aptes à être reconnus par des anticorps formés chez un hôte infecté par un rétrovirus de la classe HIV-2 et sont caractérisés en ce qu'ils présentent des propriétés immunologiques, le cas échéant immunogènes, en commun avec la séquence PF10 suivante :
XKFDDPHGETLVWEFDPLLAYSYEAFIRYPEEFGHKZ
dans laquelle X et Z ont des significations analogues aux précédentes à condition toutefois que leur choix permette la conservation de la capacité du peptide ci-dessus à être reconnu par des sérums humains contenant des anticorps contre la protéine F d'un rétrovirus HIV-2 et plus particulièrement des anticorps monospécifiques dirigés contre le peptide ci-dessus.

Un autre peptide conforme à l'invention apte à être reconnu par des anticorps formés chez un hôte infecté par un rétrovirus HIV-2 est caractérisé en ce qu'il présente des propriétés immunologiques, le cas échéant immunogènes. en commun avec la séquence PF19 suivante :
XCRGGLEGMFYSERRHKILNIYLEKEEGIIADZ
ou la séquence :
XC(acm)RGGLEGMFYSERRHKILNIYLEKEEGIIADZ
dans laquelle X et Z ont des significations analogues aux précédentes à condition toutefois que leur choix permette la conservation de la capacité du peptide ci-dessus à être reconnu par des sérums humains contenant des anticorps contre la protéine F d'un rétrovirus HIV-2 et plus particulièrement des anticorps monospécifiques dirigés contre le peptide ci-dessus.

D'autres modifications des différentes séquences d'acides aminés décrites pour la composition des peptides de l'invention, éventuellement des délétions au niveau de certains acides aminés peuvent être envisagées dès lors que la séquence modifiée formée reste capable d'être reconnue par des anticorps monospécifiques contre l'un ou l'autre des peptides concernés, présents dans un milieu biologique, notamment un sérum humain à la suite d'une infection par un rétrovirus susceptible de provoquer un SLA ou un SIDA.

On peut notamment mettre en oeuvre une partie seulement des peptides précédemment décrites dans la mesure où une séquence de plus petite taille formée conserve des propriétés immunologiques, le cas échéant immunogènes, vis-à-vis des anticorps dirigés contre la protéine F d'un rétrovirus HIV.

L'invention concerne en outre des compositions caractérisées en ce qu'elles comprennent en mélange au moins deux des peptides précédemment décrits. De telles compositions sont utilisables pour la détection dans un échantillon biologique à tester, d'anticorps formés contre la protéine F ou contre des parties de la protéine F notamment contre des fragments de peptides éventuellement présents dans la composition, à la suite d'une infection par un rétrovirus HIV, notamment HIV-1 ou HIV-2.

L'invention concerne à cet égard, des compositions pour le diagnostic non spécifique de l'infection par un rétrovirus du type HIV. De telles compositions comprennent un mélange d'au moins deux peptides différents choisis parmi les peptides PF10 à PF19 ou des variants de ces peptides définis par leur aptitude à être reconnus par des anticorps formés contre la protéine F ou contre les peptides dont ils sont dérivés.

L'invention vise également des compositions permettant le diagnostic spécifique d'une infection due à un rétrovirus humain HIV-1 ou HIV-2 et comprenant au moins deux peptides différents parmi les précédents dès lors qu'ils sont caractéristiques de la séquence de la protéine F du virus responsable de l'infection que l'on

cherche à détecter ou qu'ils présentent la propriété d'être reconnus par des anticorps formés à la suite d'une infection par ledit virus.

Des compositions particulières pour le diagnostic spécifique d'une infection par HIV-1 comprennent au moins deux peptides différents parmi les peptides PF11 à PF18 ou leurs variants définis ci-dessus.

Selon des modes de realisation particulièrement avantageux de l'invention, on mentionne des compositions comprenant en mélange :

- au moins un peptide de la région C-terminale de la protéine F, par exemple choisi parmi PF15, PF16 et PF17 et, au moins un peptide choisi dans la région N-terminale de F, par exemple choisi parmi PF11, PF12 et PF13 ou encore
- au moins les peptides PF12 et PF17 ou
- au moins les peptides PF12 et PF16 et éventuellement PF17 ou
- au moins les peptides PF11 et PF17 et éventuellement PF16
- au moins les peptides PF13 et PF17 et/ou PF16 ou encore toute autre combinaison avantageuse pour la détection d'anticorps.

L'invention concerne également dans le cadre des définitions ci-dessus des compositions comprenant un mélange de l'ensemble des différents peptides ci-dessus.

Dans un mode de réalisation particulier de l'invention, certains des peptides PF11 à PF18 ci-dessus sont mis en oeuvre dans des mélanges capables d'être reconnus par des anticorps présents dans un sérum humain infecté par un rétrovirus de la classe HIV-1.

Dans un autre mode de réalisation on réalisera un mélange des peptides PF10 et PF19, capable d'être reconnu par des anticorps présents dans un sérum humain infecté par un rétrovirus HIV-2.

On peut également réaliser des mélanges de peptides reconnus par des anticorps dirigés contre un rétrovirus du type HIV-1 d'une part et des peptides reconnus par un rétrovirus du type HIV-2 d'autre part.

L'invention a trait également à des compositions comprenant de tels peptides seuls ou en mélange avec d'autres protéines ou peptides codés par le génome d'un rétrovirus capable de causer un SLA ou un SIDA et à leur utilisation dans un procédé de détection in vitro dans un échantillon biologique, de l'infection par un rétrovirus du type HIV-1 ou HIV-2.

L'invention est également relative à la synthèse des peptides intéressants de la protéine F.

Un principe de synthèse est par exemple de mettre en présence une phase solide dans laquelle s'assemble la séquence du peptide et une phase liquide contenant solvants et réactifs. A chaque étape la séparation entre le peptide en croissance et les réactifs se fait par simples filtrations et lavages.

Initialement, le support solide qui porte un groupement réactif réagit avec le carboxyle d'un acide aminé introduit avec son $\alpha$-$NH_2$ bloqué (protégé par exemple par le t-butyloxy carbonyle), pour établir une liaison covalente. Après déprotection de la fonction aminée (par exemple en lavant avec un acide tel que l'acide trifluoroacétique), un deuxième acide aminé protégé est introduit pour former par couplage la première liaison peptidique. Le deuxième acide aminé, qui fournit le deuxième amino-acyle de la séquence, est couplé à partir du résidu amino-acyle C-terminal sur la fonction amine déprotégée du premier acide aminé C-terminal de la chaîne. De préférence la fonction carboxyle de ce deuxième acide aminé est activée, par exemple par dicyclohexylcarbodiimide. Par une suite de réactions, déprotections et couplages, la synthèse progresse du résidu C- vers le résidu N-terminal. Lorsque la séquence voulue a été assemblée le peptide est décroché de la phase solide, par une réaction spécifique de coupure de la liaison peptide- résine établie initialement. Le protocole détaillé de cette synthèse est décrit dans l'exemple 1.

Le procédé de synthèse d'un peptide conforme à ce qui est décrit plus haut est caractérisé en ce qu'il comprend les étapes suivantes :

a/ mise en contact d'un support solide portant un groupement réactif apte à réagir avec une fonction carboxyle, avec le premier acide aminé C-terminal de la chaîne à synthétiser, protégé au niveau de la fonction amine devant ultérieurement intervenir dans le couplage avec un autre acide aminé, pour éviter l'auto-condensation ;

b/ déprotection de la fonction amine de l'acide aminé précédemment fixé ;

c/ couplage de la fonction amine déprotégée de l'acide aminé à l'issue de l'étape b/ avec la fonction carboxyle d'un deuxième acide aminé, protégé au niveau de sa fonction amine et qui fournit le deuxième résidu aminoacyle alors couplé au résidu aminoacyle C-terminal du peptide à synthétiser ;

d/ déprotection de la fonction amine du deuxième acide aminé ajouté et répétition des étapes c/ de couplage et d/ de protection, successivement avec les acides aminés correspondant respectivement aux résidus aminoacyle successifs jusqu'à aboutir au résidu aminoacyle N-terminal dudit peptide ;

e/ séparation du peptide formé, par traitement de la phase solide portant le peptide synthétisé, notamment au moyen de l'acide fluorhydrique, après incorporation de l'acide-aminé N-terminal ;

f/ récupération de la séquence peptidique à partir de la solution.

Dans un mode de réalisation préféré du procédé précédent, l'étape b/ de déprotection est suivie par une étape de neutralisation destinée à rendre la fonction amine nucléophile.

Dans un autre mode de réalisation particulier, on peut encore avoir recours à une seconde étape de couplage réalisée après lavage et neutralisation du peptide obtenu à l'issue de l'étape c/, au moyen d'un agent de couplage, afin de permettre la réaction des fonctions amines qui n'avaient pas réagi lors du premier couplage.

Pour certains acides aminés tels que le tryptophane ou la cystéine, la déprotection des fonctions réactives

sera obtenue après la séparation du peptide, de la résine respectivement au moyen d'une base ou avec un traitement avec l'acétate mercurique.

Il est entendu que le procédé de synthèse des peptides selon l'invention n'est pas limité au procédé précédemment décrit mais englobe au contraire toutes les variantes dans la mesure où la séquence peptidique ainsi synthétisée conserve les propriétés immunologiques souhaitées.

Selon un autre mode de réalisation de l'invention, on aura recours à la technique de synthèse en solution homogène décrite par HOUBENWEYL dans l'ouvrage intitulé "Méthode der Organischen Chemie" (Méthode de la Chimie Organique) édité par E. Wunsch, vol. 15-I et II, THIEME, Stuttgart 1974.

Cette méthode de synthèse consiste à condenser successivement deux-à-deux les aminoacyles successifs dans l'ordre requis, ou à condenser des aminoacyles et des fragments préalablement formés et contenant déjà plusieurs aminoacyles dans l'ordre approprié, ou encore plusieurs fragments préalablement ainsi préparés, étant entendu que l'on aura eu soin de protéger au préalable toutes les fonctions réactives portées par ces aminoacyles ou fragments, à l'exception des fonctions amines de l'un et carboxyles de l'autre ou vice-versa, qui doivent normalement intervenir dans la formation des liaisons peptidiques, notamment après activation de la fonction carboxyle, selon les méthodes bien connues dans la synthèse des peptides. En variante, on pourra avoir recours à des réactions de couplage mettant en jeu des réactifs de couplage classique, du type carbodiimide, tels que par exemple la 1-éthyl-3-(3-diméthyl-amino propyl)carbodiimide. Lorsque l'aminoacyle mis en oeuvre possède une fonction acide supplémentaire (notamment dans le cas de l'acide glutamique), ces fonctions seront par exemple protégées par des groupes t-butylester.

L'invention est également relative à un procédé de détection in vitro d'une infection par un rétrovirus susceptible de provoquer un SLA ou SIDA, caractérisé en ce qu'il comprend les étapes suivantes:

a) la mise en contact d'au moins un peptide selon l'invention avec un échantillon biologique à tester, susceptible de contenir des anticorps contre la protéine F d'un rétrovirus HIV ci-dessus, dans des conditions permettant la formation d'un complexe immunologique peptide-anticorps,

b) la détection du complexe immunologique formé.

Pour réaliser la détection du complexe immunolologique peptide-anticorps et ainsi faire le diagnostic de l'infection, on pourra par exemple avoir recours à un dosage radioimmunologique. On peut également avoir recours à une méthode d'immunoenzymologie telle que la méthode ELISA.

Dans un premier mode de réalisation du test de détection d'une infection par un rétrovirus capable de provoquer un SLA ou un SIDA, le peptide mis en oeuvre pour effectuer le diagnostic de la présence d'anticorps anti-protéine F est reconnu spécifiquement par un rétrovirus HIV-1.

Dans un deuxième mode de réalisation de ce test de détection, le peptide mis en oeuvre est reconnu spécifiquement par un rétrovirus HIV-2.

Dans un autre mode de réalisation de ce test, on choisit de mettre en oeuvre un mélange de peptides reconnaissant d'une part des anticorps formés contre la protéine F d'un rétrovirus HIV-1 et des peptides reconnaissant d'autre part des anticorps formés contre la protéine F d'un rétrovirus HIV-2. On peut ainsi réaliser un diagnostic non spécifique d'une infection par l'un ou l'autre des rétrovirus HIV-1 ou HIV-2.

Pour effectuer le diagnostic le peptide pourra avantageusement être fixé sur une plaque ELISA.

L'invention concerne aussi l'utilisation des peptides de la protéine F, pour purifier des anticorps monospécifiques contre la protéine F.

Pour effectuer une telle purification, par exemple par chromatographie d'affinité, on peut opérer comme suit :

- fixation des peptides utilisés pour la purification sur un support,
- passage sur ce support d'une solution contenant les anticorps que l'on cherche à purifier, pour les amener au contact des peptides fixés, dans des conditions permettant la formation d'un complexe immunologique peptide-anticorps ; ladite solution peut être un sérum polyclonal ou un surnageant ou encore un ascite contenant des anticorps monoclonaux,
- élimination des constituants n'ayant pas réagi,
- récupération des anticorps monospécifiques dirigés contre la protéine F.

L'invention concerne également les anticorps monospécifiques dirigés contre la protéine F, caractérisés en ce qu'ils sont reconnus par un ou plusieurs peptides précédemment décrits.

L'invention vise en outre des anticorps polyclonaux et des anticorps monoclonaux, dirigés contre les peptides décrits ci-dessus. Ces anticorps anti-peptides peuvent être utilisés pour détecter la présence de la protéine F d'un rétrovirus HIV.

L'invention concerne également un kit pour le diagnostic in vitro d'une infection par un rétrovirus capable de provoquer un SLA ou un SIDA, caractérisé en ce qu'il comprend :

- un peptide conforme à l'invention, ou un mélange de ces peptides ;
- les réactifs pour la constitution d'un milieu, notamment une solution tamponnée, propice à la formation d'une réaction immunologique entre le ou les peptides et les anticorps éventuellement présents dans un échantillon biologique ;
- un ou plusieurs réactifs éventuellement marqué(s) aptes(s) à réagir avec le ou lesdits peptides pour la détection du complexe peptideanticorps formé ;
- le cas échéant, un milieu biologique de référence, tel qu'un sérum, semblable au milieu biologique provenant de la personne éventuellement infectée avec un rétrovirus HIV et dépourvu d'anticorps reconnaissant les susdits peptides.

Lorsque le réactif de diagnostic est constitué par des anticorps dirigés contre les peptides de l'invention, pour la détection de la présence de la protéine F dans un échantillon biologique testé, le kit de détection comprend :
- des anticorps dirigés contre l'un au moins des peptides ci-dessus décrits,
- les réactifs pour la constitution d'un milieu, notamment une solution tamponnée, propice à la formation d'une réaction immunologique entre le ou les anticorps du réactif et la protéine F éventuellement présente dans l'échantillon biologique,
- un ou plusieurs réactifs éventuellement marqué(s) apte(s) à réagir avec le ou lesdits anticorps pour la détection du complexe anticorps-protéine F formé,
- le cas échéant, un milieu biologique de référence, tel qu'un sérum, semblable au milieu biologique provenant de la personne éventuellement infectée avec un rétrovirus HIV et dépourvu de la protéine F reconnue par lesdits anticorps.

Les peptides peuvent être utilisés pour le traitement de la maladie due au rétrovirus HIV, après avoir subi d'éventuelles modifications permettant leur introduction à travers la membrane cellulaire et l'augmentation de leur demi-vie par exemple dans le sang. Des modifications comme la myristilation ou la transformation de peptides L en peptides D peuvent être envisagées.

De plus les peptides de l'invention peuvent être utilisés pour la vaccination dans des compositions immunogènes.

Les exemples qui suivent illustrent la synthèse des peptides selon l'invention et leur utilisation dans un test de diagnostic de la présence d'anticorps anti-protéine F contenus dans des sérums humains connus pour avoir été infectés par un virus HIV-1.

D'autres caractéristiques apparaissent aussi dans les figures.

Figure 1 : représente l'alignement des séquences d'acides aminés de la protéine F pour les isolats ROD.P de HIV-2 et LAV.P de HIV-1.

Figure 2 : représente les relations entre le rendemenent global d'incorporation des acides aminés et la pureté des produits obtenus par synthèse en phase solide (d'après Wang, S.S., 1972).

Figure 3 : représente la protéine F de 4 isolats du rétrovirus HIV-1.

Figure 4 : Structure primaire selon le code à une lettre de la protéine F (nef) de l'isolat HIV-1 Bru. Les séquences peptidiques synthétisées chimiquement sont soulignées par des flèches. On note que la variabilité de la protéine F d'une souche HIV à une autre est d'environ 2 à 17%.

Figure 5 : Radioimmunoessai en phase liquide de $^{125}I$ nef avec deux sérums humains HIV positifs (courbes A et B). La courbe de liaison de $^{125}I$ nef est donnée en fonction de la dilution du sérum. Un taux de 50% de liaison est obtenu avec une dilution de sérum proche de 1:200 et 1:500. Les liaisons non spécifiques de $^{125}I$ nef sont évaluées en utilisant deux sérums HIV-négatifs (courbes C et D).

Figure 6 : Radioimmunoessai en phase liquide de $^{125}I$ nef avec un sérum anti-nef-positif humain (1:200). La spécificité de liaison de $^{125}I$ nef est démontrée par la capacité de la protéine nef non radioactive d'inhiber la liaison des anticorps anti-nef à la protéine $^{125}I$ nef. La concentration de nef capable d'inhiber 50% de liaisons de protéine $^{125}I$ nef aux anticorps anti-nef (c'est-à-dire $K_{0,5}$) est 2,2 x $10^{-9}$ M.

## EXEMPLE 1

## SYNTHESE, PURIFICATION ET CARACTERISATION DES PEPTIDES

### 1/Nature du suport solide :

Pour la réalisation de la synthèse, un bon support solide est choisi de fa on à répondre aux conditions suivantes :
- être insoluble dans les solvants et réactifs de la phase liquide ;
- être inerte vis-à-vis des réactifs de la synthèse;
- être suffisamment stable physiquement pour être agité et subir les filtrations ;
- assurer une excellente diffusion des réactifs pour ne pas ralentir la cinétique d'un facteur trop important comparé à celle des réactions en solution.

Deux sortes de résines ont été utilisées pour les besoins de la synthèse :
- une résine obtenue par copolymérisation de styrène en présence de 1% de divinylbenzène, dont la fonction réactive est le groupement benzydrylamine (-O-CHO-$NH_2$); avec une substitution de 0,2 mmole $NH_2$/g de résine ;
- une résine polyacrylamide macroporeuse dont la fonction réactive est le groupement amino méthyl (-$CH_2$-$NH_2$) à 0,5 ou 0,7 mmole $NH_2$/g).

### 2/Nature des acides aminés :

a/ Protection des groupements α-aminés.

La fonction α-$NH_2$ de l'acide aminé ajouté a été protégée pour éviter la réaction d'auto-condensation des acides aminés. Le groupement protecteur utilisé pour cette synthèse est le tertiobutyloxycarbonyle (Boc) qui

est éliminé par l'acide trifluoroacétique TFA) 30% dans le dichlorométhane (DCM).

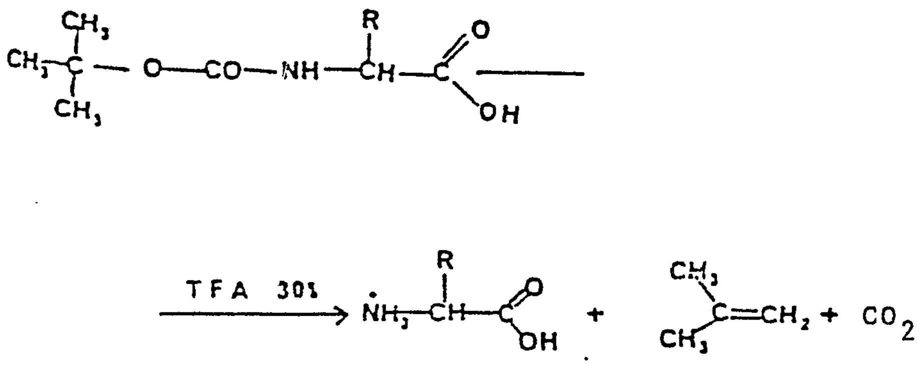


b/ Protection des groupements fonctionnels latéraux.

Contrairement à une protection temporaire des fonctions α-aminés, les fonctions portées par les chaînes latérales doivent être maintenues protégées durant toute la synthèse et, par conséquent, les protections doivent être non labiles en milieu TFA 30%. Par contre, elles seront déprotégées, en général, par le réactif utilisé pour couper la liaison peptide-résine, l'acide fluorhydrique. Les différents groupements protecteurs correspondant aux acides aminés utilisés sont représentés dans le tableau 1.

| Fonction protégée | Groupement protecteur | |
|---|---|---|
| OH de la tyrosine | 2,6-dichlorobenzyl | 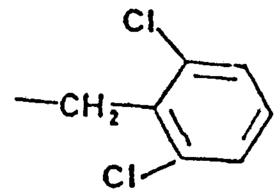 |
| OH de la thréonine<br>OH de la sérine<br>- COOH de l'aspartate<br>- COOH du glutamate | benzyl | 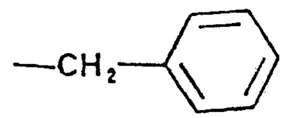 |
| SH de la cystéine | t. butylmercapto | 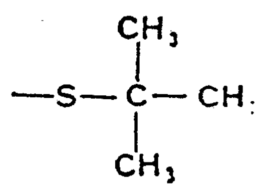 |
| imidazole de l'histidine<br>-NH de l'arginine | tosyl | 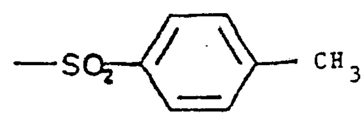 |
| $-NH_2$ de la lysine | benzyloxycarbonyl | 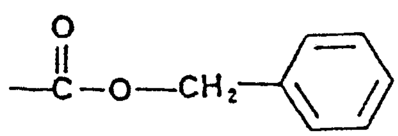 |
| Indole du Tryptophane | formyl | —CHO |

Tableau 1 : Protection des fonctions tertiaires des acides aminés utilisés dans nos synthèses.

3/Dispositif expérimental :

L'assemblage des peptides a été réalisé automatiquement dans un appareil de synthèse (APPLIED BIOSYSTEMS (marque déposée) Peptides Synthesizer modèle 430A) suivant la méthode générale décrite par MERRIFIELD).

4/Protocole d'incorporation d'un résidu:

Le protocole expérimental permettant d'effectuer un cycle de synthèse comporte les étapes suivantes :

a/-Déprotection des fonctions aminées

Elle est obtenue après 2 prétraitements de 2 minutes suivis d'un traitement de 30 minutes avec 30% de $TFA/CH_2Cl_2$. Les conditions utilisées sont normalement suffisantes pour éliminer complètement les groupements Boc protecteurs des fonctions $\alpha$-$NH_2$,

b/-Lavage

Les produits déprotégés ont été lavés 5 fois 2 minutes avec $CH_2Cl_2$.

c/-Neutralisation

Nécessaire pour désioniser l'amine $\alpha$-$NH_2$ du peptide sur la résine et la rendre nucléophile pour participer à la réaction de couplage, la neutralisation est obtenue par 2 prétraitements de 2 minutes suivis d'un traitement de 10 minutes avec 5% de $DIEA/CH_2Cl_2$ (le DIEA étant le Di-Isopropyl-Ethyl-Amine).

d/-Premier couplage

Le premier couple est réalisé dans un excès (3 fois) de Boc acide aminé (aa) et du dicyclohexylcarbodiiodiimide (DCC) dans $CH_2Cl_2$ (2 heures). Lors du couplage dont le mécanisme est représenté ci-après, une réaction secondaire donne un dérivé uréique non réactif qui dépend de la nature du solvant.

Cette réaction est assez faible dans le dichlorométhane et elle est contrebalancée par l'utilisation d'un excès de réactifs (DCC et Boc-a.a).

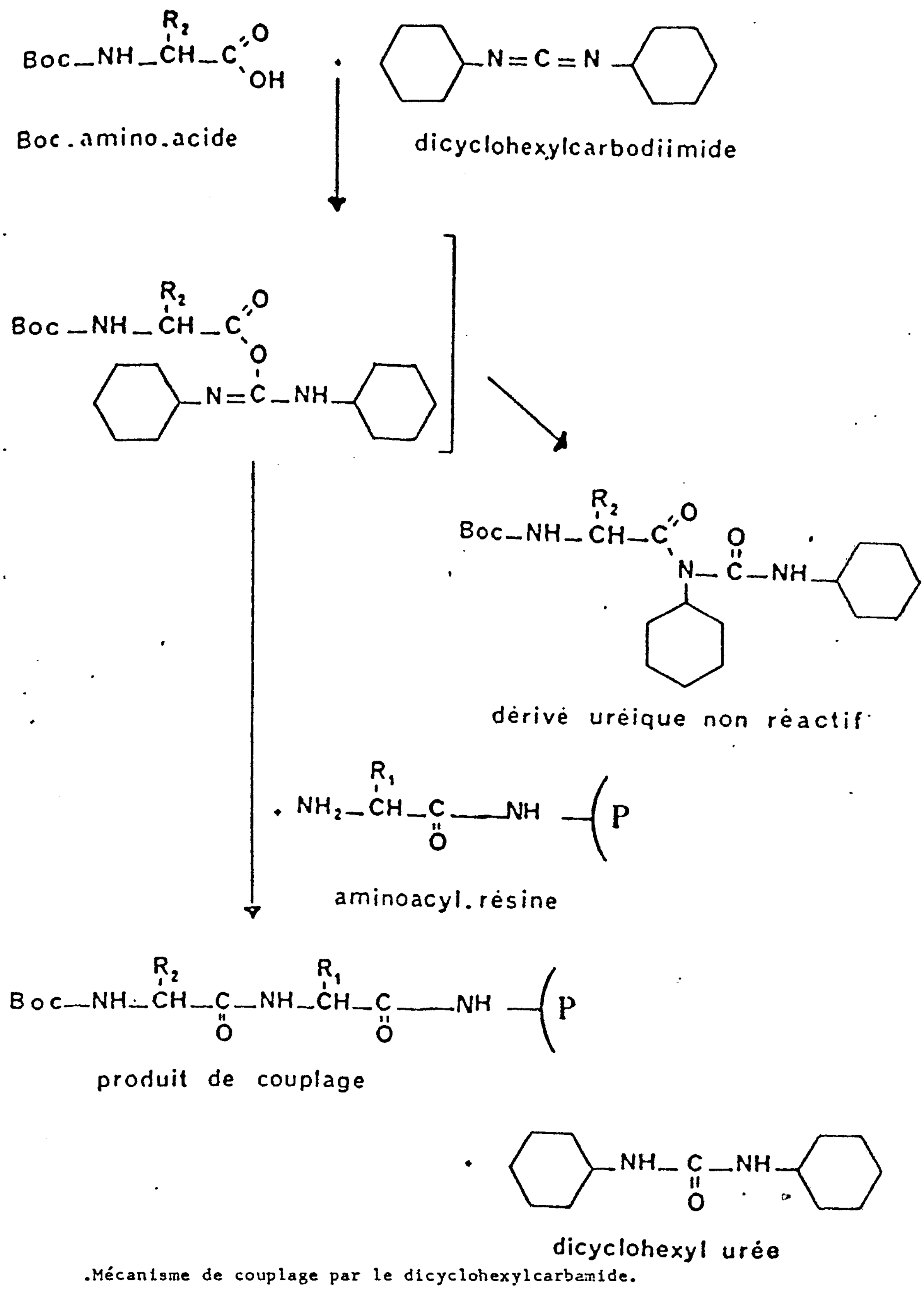

Boc.amino.acide

dicyclohexylcarbodiimide

dérivé uréique non réactif

aminoacyl.résine

produit de couplage

dicyclohexyl urée

.Mécanisme de couplage par le dicyclohexylcarbamide.

e/-Lavage

On lave le produit de réaction 5 fois, 2 minutes avec $CH_2Cl_2$.

f/-Nouvelle neutralisation

Pour réaliser cette nouvelle neutralisation, on fait 2 prétraitements de 2 minutes suivis d'un traitement de 10 minutes avec 5% de DIEA/$CH_2Cl_2$.

Cette nouvelle neutralisation permet l'activation des α-amines qui auraient échappé au premier traitement basique.

g/-Lavage

On lave le produit 5 fois 2 minutes avec $CH_2Cl_2$ et 1 fois 2 minutes avec la diméthylformamide (DMF).

h/ 2ème couplage

Ce deuxième couplage fait intervenir comme réactif l'ester de benzotriazole du Boc: acide aminé 1,5 fois en excès dans la DMF (1 heure).

L'ester de benzotriazole du Boc (aa) est préparé extemporanément comme suit :
- on prépare un mélange équimoléculaire de DCC et d'hydroxybenzotriazole (HOBt) dans la diméthylformamide (DMF) et on laisse incuber pendant dix minutes à 0°C. Puis, le Boc acide aminé est ajouté et la réaction est poursuivie à 0°C pendant dix minutes. L'ester de benzotriazole du Boc acide aminé ainsi formé est ajouté dans le vase à réaction.

i/-Lavage

On a à nouveau lavé le produit 5 fois 2 minutes avec $CH_2Cl_2$.

5/Contrôles analytiques en cours de synthèse:

Du fait que la synthèse se déroule sans purification des intermédiaires, il est impératif de contrôler que les réactions de couplages sont complètes. Le diagramme de la figure 2 montre l'importance d'avoir des couplages complets pour la synthèse des grandes séquences ; en effet si l'on synthétise un peptide de 40 résidus avec un rendement global (déprotection, neutralisation et couplage) de 98%, on obtient un mélange contenant 46% du peptide voulu et 54% de différents peptides plus petits. Si le rendement est de 99% en moyenne par étape le peptide entier sera présent à 67%. Si le rendement est de 99,5% le peptide voulu représententera alors 82% et seulement 18% de mélanges de peptides plus petits.

Pour vérifier la bonne réalisation du couplage, trois tests de couplages peuvent être mis en oeuvre :
- Test à la ninhydrine (Kaiser,et coll.,1970) : on prélève une partie aliquote de peptidyl-résine (environ 10mg) qu'on lave bien à l'éthanol dans des petits tubes en verre. Après centrifugation et élimination de l'éthanol, on ajoute une solution de ninhydrine et on laisse réagir pendant 5 minutes à 95°C.

L'apparition d'une coloration bleue (test positif) signifie un couplage incomplet. Si la couleur de la résine ne change pas le couplage a eu lieu au moins à 99%.
- Test à la fluorescamine (Felix et Jimenez, 1973) : un peu plus sensible que celui à la ninhydrine, il permet de détecter moins de 1% de $NH_2$ libre. Une partie aliquote de peptidyl-résine (10 à 20mg) est lavée successivement au DCM, à l'éthanol et au DIEA 5% dans le DCM puis on ajoute la fluorescamine. La réaction a lieu dans un milieu basique (DIEA 5%) pendant 10 minutes. Après lavage et sèchage, on regarde la résine sous une lampe UV à une longueur d'onde de 366nm. L'apparition d'une fluorescence signifie la présence de $NH_2$ libres.
- Test au chloranyl (Christensen, 1979) : après le couplage d'un acide aminé quelconque sur une proline on ne peut utiliser les deux premiers tests à cause de leur faible sensibilité dans la détection des amines secondaires. On utilise le chloranyl (2,3,5,6 tétrachloro 1,4 benzoquinone). A une partie aliquote de peptidyl-résine (5 à 10mg) on ajoute 200 µl d'acétone et 50 µl d'une solution saturée de chloranyl dans le toluène; le tout est maintenu en agitation pendant 5 minutes. L'apparitioion d'une coloration verte signifie un couplage incomplet. Selon le résultat du test, s'il demeure positif après un second ou troisième couplage, on bloque par acétylation les fonctions α-$NH_2$ qui n'ont pas réagi pour éviter la formation de peptide à délétion, ou bien, s'il est négatif un nouveau cycle de déprotection-couplage est entamé. Par ailleurs, on titre à intervalles réguliers (tous les deux ou trois résidus incorporés) la quantité d'amines présentes sur la résine. Cette quantité doit rester constante et égale à la quantité d'acide aminé fixée initialement sur la phase solide. On utilise pour cela la méthode des sels d'acide picrique (Gisin, 1972) : 10 à 20 mg de peptidyl-résine séchés sont pesés avec précision dans une seringue et traités de la façon suivante :
- 5 lavages au DCM
- 3 traitements de 3 minutes avec une solution d'acide picrique (0,01 M dans DCM)
- 5 lavages au DCM pour éliminer l'excès d'acide picrique
- 5 traitements de 5 minutes avec la DIEA (5% dans DCM) qui déplacent le picrate lié à la résine. On recueille les filtrats dans une fiole jaugée de 100 ml.
- lavage de la résine avec de l'éthanol puis on complète la fiole à 100 ml avec l'éthanol
- mesure de la densité optique à 358 nm : sachant la valeur de l'εM (coefficient d'extinction molaire) 358 nm (13800) on calcule la concentration des α-$NH_2$ libres qui ont pu réagir avec l'acide picrique pour former le sel

correspondant.

6/Acétylation du peptide sur la résine :

Si le couplage d'un acide aminé demeure incomplet après plusieurs tentatives de réaction, ou si la synthèse du peptide est terminée et que l'on désire bloquer l'extrémité α-$NH_2$ du peptide par le groupement acétyl (l'acétylation de l'extrémité N-terminale est faite dans l'objectif d'avoir un peptide qui mime au point de vue de la charge, celle qui existe dans la protéine native), le peptide sur la résine est acétylé dans les conditions suivantes :

- neutralisation par 5% DIEA
- lavages DCM
- lavages DMF
- acétylation par le mélange d'1ml d'anhydride acétique (10 mmoles) et de 1,7 ml de DIEA (10 mmoles) dans la DMF. La réaction dure 20 à 30 minutes et est considérée comme complète lorsque le test de couplage est négatif.

7/Coupure de la liaison peptide-résine :

La rupture de la liaison peptide-phase solide est faite par l'acide fluorhydrique anhydre (HF) à 0° C (Lenard et Robinson, 1967 ; Sakakibara etcoll., 1967).

Elle s'accompagne, si on a choisi des protecteurs labiles dans l'HF, de la déprotection des chaînes latérales. La présence d'anisole (10% en volume) est nécessaire pour piéger les radicaux libres et éviter ainsi l'alkylation de certains résidus, comme la tyrosine, par les groupements protecteurs libérés. En utilisant une résine benzhydrylamine on obtient, à l'issue de la réaction, le peptide sous sa forme C-α amidée. Après évaporation de l'acide fluorhydrique, l'anisole est extraite par l'éther et le peptide est solubilisé par l'acide acétique. Le rendement de la réaction de coupure est calculé à partir des résultats d'analyses d'acides aminés donnant les quantités de peptide avant (peptide fixé à la résine) et après (peptide brut libre) la coupure par l'acide fluorhydrique. Cependant certains groupements protecteurs des chaînes latérales de quelques acides aminés, comme le formyl tryptophane ne sont enlevés que par un traitement supplémentaire.

8/Déprotection du tryptophane :

Sous la forme formyl tryptophane, l'acide aminé aromatique est convenablement protégé durant la synthèse contre les risques d'oxydation du groupement indole. Ce groupement indole peut être régénéré par action d'une base (1 M hydroxylamine, pH 9) pendant une heure en milieu aqueux (Ohno et coll., 1972).

Les peptides qui ont été synthétisés sont caractérisés par des propriétés physico-chimiques données dans le tableau 3 suivant :

| HIV-1 Localisation dans la séquence de la protéine F représentée figure 2 | Masse Molaire en g. | Masse Molaire avec grps. de protection en g. | ε Théorique à 280 nm en $cm^{-1}M^{-1}$ | Charge nette à pH neutre | Homologie avec la séquence correspondante de HIV-2 en % |
|---|---|---|---|---|---|
| PF11 1-31 | 3636 | 5546 | 12350 | +3 | 26 |
| PF12 1-66 | 7015 | 10333 | 16650 | -1 | 23 |
| PF13 32-64 | 3578 | 5281 | 5550 | -4 | 18 |
| PF14 65-109 | 5222 | 7480 | 1750 | +4 | 58 |
| PF15 118-167 | 5886 | 8610 | 16600 | pour forme réduite -1 | 56 |
| PF16 171-205 | 4408 | 6225 | 7550 | 0 | 40 |
| PF17 141-205 | 7830 | 11769 | 9050 | +1 | 38 |
| PF18 93-122 | 3735 | 5564 | 8300 | +1 | 40 |
| PF19 de HIV-2 125-154 | 3714 | 5756 | 2750 | 0 | avec HIV-1 40% |

Figure 3

EXEMPLE 2

ETUDE SEROLOGIQUE AVEC LE PEPTIDE PF 16 LA PROTEINE F OBTENU PAR SYNTHESE COMPARATIVE

Le peptide a été préalablement synthétisé par la méthode décrite précédemment. Ce peptide couvre la séquence de 35 acides aminés de la partie C-terminale de la séquence d'acides aminés de la protéine F du rétrovirus HIV-1.

Le peptide PF16 a la séquence d'acides aminés suivante, dans laquelle l'acétamido-méthyle (acm) représente un groupe hautement protecteur des fonctions SH des cystéines :
GMDDPEREVLEWRFDSRLAFHHVARELHPEYFKNC(acm)

Pour les besoins du test, les différents peptides PF16, PF19, PF13, PF17 utilisés sont dilués à une concentration de 2 mg/ml.

Sur une bande de papier BIORAD pour transblot, on dépose différents spots : 1µl, 2µl, 5µl, 10µl.

Après séchage on pratique un test Western Blot classique en utilisant comme anticorps des sérums de patients infectés par le virus HIV-1, positifs pour la protéine F, à des dilutions variant de 1/100 à 1/300.

Les peptides testés permettent d'aboutir aux résultats résumés dans le tableau 2 avec trois sérums différents anti-F + + (+ + signifiant une réaction plus marquée).

Par ailleurs, les inventeurs ont constaté qu'un anticorps, monoclonal anti-protéine F d'un rétrovirus HIV-1 produit chez la souris reconnait le peptide PF16 de l'invention dans un test ELISA.

Ceci montre bien le caractère immunodominant des peptides selon l'invention.

TABLEAU 2

| | SERUMS POSITIFS, ANTI-PROTEINE F DE HIV-1 | | | | | | | | | | | | | | | |
|---|---|---|---|---|---|---|---|---|---|---|---|---|---|---|---|---|
| | CHA 1/300 | | | | P.6. 1/200 | | | | 1545 1/200 | | | | LOM 1/100 | | | |
| PEPTIDE | 10µl | 5µl | 2µl | 1µl | 10µl | 5µl | 2µl | 1µl | 10µl | 5µl | 2µl | 1µl | 10µl | 5µl | 2µl | 1µl |
| PF 16 | ++ | ++ | ++ | ++ | + | - | - | - | ++ | - | - | - | ++ | ++ | + | + |
| PF 19 | ++ | ++ | - | - | + | tr | - | - | + | + | - | - | tr | tr | - | - |
| PF 13 | + | + | tr | tr | - | - | - | - | tr | - | - | - | - | - | - | - |
| PF 17 | - | - | - | - | - | - | - | - | tr | - | - | - | + | + | + | + |

EXEMPLE 3

- Utilisation de peptides synthétiques pour la détection d'anticorps dirigés contre la protéine de régulation nef dans des sérums de patients infectés par HIV.

On rappelle à toutes fins utiles que la désignation "nef" est une autre désignation donnée à la protéine F (encore appelée 3' orf, B, E').

Cet exemple concerne la détection d'anticorps anti-nef dans des sérums HIV positifs, au moyen de radioimmuno-essais avec un organisme recombinant synthétisant la protéine nef ; cet exemple concerne également une étude de l'antigénicité de longs fragments synthétiques de nef, en vue de localiser les régions immunodominantes de la molécule et de caractétiser les anticorps anti-nef dans des sérums humains exposés à HIV.

MATERIELS ET METHODES

- Tests immunologiques

Des bactéries recombinantes E.coli contenant le gène nef ont été utilisées. Ces bactéries sont décrites par Guy B et al. Nature 1987,330:266-269. 300 sérums humains de patients infectés ont été utilisés pour la réalisation de ces tests. Les complexes IgG de souris anti-humaines -(H+L)- peroxydase et o-phenylendiamine (OPD) ont été obtenus auprès de Pasteur Diagnostic (France), le constituant $^{125}I$ Na est un produit Amersham (Grande Bretagne) la protéine A-Sephérose et la Colonne de Sephadex $G_{25}$ $PD_{10}$ sont des produits de Pharmacia (Uppsala, Suède), des plaques de microtitration à 96 puits proviennent de Nunc (Rotskild, Danemark) et l'iodogène provient de Pierce Chermicals (Rockford, IL, USA). Le compteur gamma et le photomètre titertek Multiskan MCC 340 proviennent respectivement de Kontron Instruments (Zurich, Suisse) et de Flow laboratories (Uppsala, Suède).

- Synthèse chimique de peptide

Cette synthèse a été réalisée suivant le même protocole que celui décrit dans l'exemple 1 et faisait donc appel aux mêmes produits.

- Production de la protéine nef

La protéine nef a été synthétisée dans des bactéries E.Coli de la manière décrite dans l'article de Guy, B. et al. La protéine nef obtenue à partir de E.coli n'est pas myristylée. Les protéines de cellules lysées E.coli ont été extraites au moyen d'urée en présence de benzamidine. Après dialyse et centrifugation, le surnageant a été chromatographié sur bleu Cibracron. La fraction enrichie en nef a finalement été purifiée par ultrafiltration. L'homogénéité de la protéine nef, déterminée par la méthode HPLC était d'environ 75 à 90%.

- Iodation de nef

On a ajouté 10µg de nef dans 50µl de tampon phosphate salin (PBS) à PH 7,4 et avec 0,5 mCi de $^{125}I$ Na (13-17 mCi/µg) à un tube pré-recouvert avec 60 nmoles de iodogène. Cette préparation a été incubée pendant 10mn à température ambiante. La réaction a été arrêtée par addition de 10µl de tyrosine (9mg/ml). La protéine iodée a été débarrassée de $^{125}I$ Na par filtration sur une colonne de Sephadex G25 (PD 10) équilibrée avec un tampon PBS - 0,5% de sérumalbumine bovine (BSA). La radioactivité spécifique de nef marquée par $^{125}I$ nef était approximativement de 25 µCi/µg.

- Radioimmunoessai (RIA)

50 µl de $^{125}I$ nef (60.000 cpm) ont été incubés avec 50µl de sérum à des dilutions variées pendant 2 heures à 37° C. Les complexes préformés entre $^{125}I$ nef et les anticorps anti-nef ont été immunoprécipités avec 100 µl d'une suspension de protéine A-Sepharose dans un tampon PBS à pH 7,4 avec 0,5% de BSA (1v/2v) pendant 1 heure à 37° C. Après deux lavages avec le tampon PBS à pH 7,4 conterant 0,5% de BSA, 0,05% de $NaN_3$, 0,01% de tween 20 (marque déposée) la radioactivité de liaison a été comptée sur un compteur gamma.

- Test ELISA pour la détection d'anticorps anti-nef.

Des plaques de microtitration à 96 puits ont été recouvertes une nuit avec 500 ng de protéine nef bactérienne, à 37° C ou avec 500 ng de peptides synthétiques par puits dans 50µl de PBS à pH 7,4. Après saturation avec 400 µl de PBS pH 7,4, contenant 5% de caséine, durant 1 heure à 37° C et lavage avec PBS à pH 7,4, 0,5% de BSA, 0,01% tween, 50 µl de sérums à différentes dilutions ont été ajoutés pendant 2 heures à 37° C. Après le rinçage, 50µl de complexes d'IgG de souris anti-humaine-(H + L)-peroxydase 1:1000 ont été incubés pendant 1 heure à 37° C. Après un nouveau rinçage, 100µl de OPD ont été ajoutés pendant 30mn à température ambiante dans l'obscurité. La réaction de l'enzyme a été arrêtée par addition de 50µl d'acide sulfurique 4N et la densité optique des plaques a été lue à 492 nm/620 nm. Les plaques recouvertes avec la caséine ont été utilisées comme contrôle négatif.

RESULTATS

- Détection d'anticorps d'anti-nef par RIA.

L'immunoréactivité de la protéine F marquée avec $^{125}I$ avec les anticorps anti-nef a été testée par la méthode RIA en utilisant des sérums HIV-positifs. La figure 5 correspond à la courbe de liaison de l'anticorps à la protéine nef marquée, en fonction de la dilution du sérum. Un taux de liaison de 50% a été obtenu avec une dilution du sérum voisine de 1:200. L'existence de liaisons non spécifiques a été évaluée en utilisant des sérums sélectionnés au hasard, choisis parmi les sérums HIV-négatifs. La spécificité de la liaison avec la protéine nef marquée a été démontrée par la capacité de la protéine nef native non radioactive d'inhiber la formation du complexe immun de la protéine nef marquée par $^{125}I$ avec les anticorps anti-nef de l'un des sérums sélectionnés au hasard.

Dans ce cas la concentration de nef capable d'inhiber 50% des liaisons entre les anticorps anti-nef et la protéine nef $^{125}I$ (correspondant à $K_{0,5}$) était 2,2 x $10^{-9}$ M (figure 6).

- Prévalence des sérums anti-nef positifs chez les Patients infectés par HIV.

Afin d'estimer le pourcentage (%) de sérums anti-nef-positifs parmi des patients infectés avec HIV, 300 sérums HIV-positifs et 100 sérums HIV-négatifs (correspondant à un contrôle négatif) ont été testés pour détecter la présence d'anticorps anti-nef par la méthode RIA. Les essais ont été réalisés sur des sérums dilués à 1:200 en présence de protéine nef marquée avec $^{125}I$ (60.000 cpm). Les liaisons non spécifiques détectées, des sérums négatifs de contrôle avec la protéine nef marquée correspondaient à des valeurs d'environ 300 ± 50 cpm comparées à des valeurs d'environ 1500 à 6000 cpm pour des sérums positifs. 211 parmi les 300 sérums HIV-positifs testés présentaient une immunoréactivité significative avec la protéine nef marquée (70 ± 5,3% (95% d'intervalle de confiance)).

- Utilisation de fragments peptidiques synthétiques de la protéine nef pour la détection d'anticorps anti-nef dans les sérums de patients infectés par HIV.

En vue d'étudier la possibilité d'utiliser des peptides synthétiques en tant qu'antigènes pour la détection d'anticorps anti-nef chez des patients infectés par HIV, 8 peptides synthétiques couvrant la totalité de la séquence de la protéine nef de HIV-1 (figure 4) ont été utilisés dans un test ELISA avec 31 sérums anti-nef positifs et 2 sérums anti-nef négatifs utilisés comme contrôle. Les recombinants nef ont été utilisés comme contrôle positif dans les tests ELISA et la caséine a été utilisée comme contrôle négatif. Les résultats de ces expériences sont donnés dans le tableau 4 qui illustre l'essai suivant:

Essai ELISA sur 31 sérums anti-nef-positifs et 2 sérums anti-nef- négatifs, soit pré-recouverts avec des peptides synthétiques (PF 11 à PF 18), soit en utilisant nef, la caséine ou un mélange de peptides PF 12 et PF 17. Les sérums ont été sélectionnés au hasard parmi des sérums anti-nef positifs et anti-nefs négatifs testés par la méthode RIA en utilisant $^{125}I$ nef. L'intensité de l'immunoradioactivité est notée "-" pour une densité optique (OD) inférieure à 0,2 ; "+" pour OD = 0,2 à 0,4 ; "++" pour OD = 0,4 à 0,8 et "+++" pour OD = 0,8 à 2,0.

Ces résultats peuvent être résumés comme suit :

- Les 31 sérums anti-nef positifs dans le test RIA ont présenté une réaction positive dans le test ELISA contre la protéine recombinante nef,
- Les deux sérums qui étaient anti-nef négatifs dans le test RIA ont donné une réaction négative dans le test ELISA, contre la protéine nef et contre tous les peptides synthétiques,
- 26 sérums anti-nef positifs ont répondu contre les peptides C-terminaux PF 16 et PF 17,
- 16 sérums anti-nef positifs ont réagi avec les peptides N-terminaux PF 12 et PF 11 et/ou PF 13,
- le peptide PF 14 a donné une réaction positive forte avec 3 sérums alors que les peptides PF 15 et PF 18 ont réagi avec 4 et 10 sérums respectivement,
- les 31 sérums anti-nef positifs ont fortement réagi avec un mélange de peptides N- et C-terminaux (PF 12 et PF 17).

DISCUSSION

La réplication de HIV dépend de plusieurs facteurs de régulation. Afin de mieux connaître la réponse immune de l'hôte envers les protéines HIV, la réponse humorale contre la protéine nef a été étudiée, d'une part en réalisant un test sur la spécificité d'anticorps anti-nef et d'autre part, en testant la réactivité d'anticorps anti-nef avec des peptides synthétiques.

Pour détecter des anticorps anti-nef dans les sérums de patients infectés par HIV, un test RIA a été réalisé avec un recombinant $^{125}I$ nef. La spécificité de liaison des anticorps anti-nef à la protéine nef marquée avec $^{125}I$ a été démontrée par la capacité de la protéine nef native d'inhiber de façon dosedépendante, la formation de complexes $^{125}I$ nef-anti-nef. Pour le sérum testé, il a été démontré dans les conditions expérimentales ci-dessus que la concentration en protéine nef native capable d'inhiber la liaison de 50% de protéine $^{125}I$ nef-anti-nef était d'environ 2,2 x $10^{-9}$ M. Cette valeur ($K_{0,5}$) indique l'interaction hautement affine entre l'antigène recombinant et les anticorps-anti-nef. Cependant, les titres en sérum, définis comme la dilution correspondant à un taux de 50% de liaison de protéine $^{125}I$ nef aux anticorps, étaient compris entre 1:100 et 1:1000.

Pour étudier l'utilisation des peptides synthétiques en tant qu'antigène pour un test d'immunodiagnostic et

pour étudier parallèlement la spécificité des anticorps anti-nef contenus dans des sérums infectés par HIV, 8 fragments peptidiques de la protéine nef se chevauchant ont été synthétisés par la méthode en phase solide, ces peptides étant choisis pour couvrir la totalité de la protéine nef de HIV-1. Les structures secondaires de nef ont été étudiées suivant la méthode de Chou & Fasman (Ann.Rev.Biochem.1978,47:251-276). L'un des peptides (PF 18) a été considéré comme incluant un site de liaison GTP potentiel de la protéine nef.

L'analyse de 31 sérums anti-nef positifs dans un test ELISA avec ces peptides synthétiques a montré l'hétérogénéité des épitopes reconnus. Par exemple, environ la moitié des sérums reconnaissaient exclusivement la partie C-terminale de nef (PF 15, PF 16 et PF 17) alors que 4 sérums réagissaient seulement avec la région N-terminale (peptides PF11, PF12 et PF13). Tous les sérums anti-nef positifs ont réagi fortement avec soit les peptides N- et/ou C-terminaux ce qui démontre une immunodominance de ces deux régions de la molécule. Des sérums réagissant avec le peptide PF12 (séquences 1-66) reconnaissaient les peptides PF 11 (séquence 1-31) et/ou PF 13 (séquences 32-64), indiquant qu'au moins 2 épitopes sont localisés au niveau des séquences des peptides PF 11 et PF 13 respectivement. L'analyse des propriétés hydrophiles de la protéine nef conformément à la méthode de Hopp & Woods (Proc. Natl. Acad. Sci. USA. 1981,72:3824-3828) a montré l'existence de deux régions hydrophiles potentielles sur les séquences correspondantes. Parallèlement les sérums régissant avec PF 17 (séquence 141-205) réagissaient également avec le peptide PF 16 (séquence 171-205). Le profil hydrophile de nef a montré que le peptide PF 17 comprend deux pics hydrophiles, l'un d'entre-eux correspondant au peptide antigénique PF 16 et l'autre étant inclus dans le peptide PF 15. Compte tenu de la réponse du peptide PF 16, le site antigénique majeur contenu dans le peptide PF 17 est probablement localisé dans le peptide PF 16 C-terminal. Les trois autres régions de nef correspondant au peptide PF 11 (séquences 1-31), PF 14 (séquences 65-109) et PF 18 (séquences 93-122) comprennent également des sites antigéniques. Le peptide PF 18 qui est supposé contenir le site de liaison GTP potentiel, présente de façon remarquable une immunoréactivité significative avec environ 1/3 des sérums anti-nef positifs. En prenant en considération l'immunodominance des régions N- et C-terminales de nef, la capacité d'un mélange de peptides PF 12 et PF 17 à être reconnu par des sérums anti-nef positifs a été testée. Les 31 sérums anti-nef positifs ont réagi fortement avec le mélange peptidique. Ces données montrent clairement que les épitopes majeurs sont localisés près des parties N- et C-terminales de la protéine F.

Tableau 4

| Serum | PF11 | PF12 | PF13 | PF14 | PF15 | PF16 | PF17 | PF18 | *nef* | casein | PF12 + PF17 |
|---|---|---|---|---|---|---|---|---|---|---|---|
| 1 | - | + | + | - | - | +++ | +++ | - | ++ | - | +++ |
| 2 | - | - | - | - | - | +++ | +++ | - | +++ | - | +++ |
| 3 | - | +++ | +++ | +++ | - | + | + | - | ++ | - | +++ |
| 4 | - | + | + | - | - | +++ | +++ | - | ++ | - | +++ |
| 5 | - | - | - | - | + | +++ | +++ | + | +++ | - | +++ |
| 6 | - | - | - | - | + | +++ | +++ | + | ++ | - | +++ |
| 7 | ++ | +++ | ++ | - | - | - | - | - | +++ | - | +++ |
| 8 | - | +++ | ++ | - | - | - | - | - | ++ | - | +++ |
| 9 | - | +++ | ++ | - | - | - | - | - | +++ | - | +++ |
| 10 | - | - | - | - | - | + | ++ | - | ++ | - | ++ |
| 11 | ++ | ++ | - | - | - | + | + | - | ++ | - | ++ |
| 12 | + | ++ | - | - | - | - | - | - | ++ | - | ++ |
| 13 | - | - | - | - | - | ++ | ++ | - | ++ | - | ++ |
| 14 | - | ++ | ++ | ++ | - | - | - | - | ++ | - | ++ |
| 15 | - | - | - | - | - | +++ | +++ | - | +++ | - | +++ |
| 16 | - | - | - | - | - | +++ | ++ | - | + | - | ++ |
| 17 | - | - | - | - | - | ++ | ++ | - | ++ | - | ++ |
| 18 | - | - | - | - | - | +++ | +++ | ++ | ++ | - | +++ |
| 19 | +++ | +++ | - | - | ++ | +++ | +++ | + | +++ | - | +++ |
| 20 | - | - | - | - | - | ++ | ++ | + | + | - | ++ |
| 21 | + | ++ | - | ++ | - | +++ | +++ | +++ | ++ | - | +++ |
| 22 | - | - | - | - | ++ | +++ | +++ | +++ | ++ | - | +++ |
| 23 | - | - | - | - | - | ++ | ++ | - | ++ | - | ++ |
| 24 | - | - | - | - | - | +++ | +++ | ++ | ++ | - | +++ |
| 25 | +++ | +++ | - | - | - | +++ | +++ | + | +++ | - | +++ |
| 26 | +++ | +++ | - | - | - | +++ | ++ | - | +++ | - | +++ |
| 27 | - | - | - | - | - | + | + | - | + | - | + |
| 28 | + | + | - | - | - | ++ | ++ | - | + | - | ++ |
| 29 | + | ++ | ++ | - | - | ++ | ++ | - | ++ | - | ++ |
| 30 | - | - | - | - | - | ++ | +++ | +++ | +++ | - | +++ |
| 31 | +++ | +++ | - | - | - | +++ | ++ | - | + | - | +++ |
| 32 | - | - | - | - | - | - | - | - | - | - | - |
| 33 | - | - | - | - | - | - | - | - | - | - | - |

**Revendications**

1. Peptide capable de former un complexe immunologique avec des anticorps anti-protéine F d'un rétrovirus susceptible de provoquer un SLA ou un SIDA, caractérisé en ce qu'il comporte au plus 70 aminoacides formant une séquence d'aminoacides correspondant à une partie de la protéine F d'un HIV ou à une variante de cette séquence néanmoins telle qu'elle soit immunologiquement reconnue par des anticorps anti-protéine F de rétrovirus HIV.

2. Peptide selon la revendication 1, caractériactérisé par tout ou partie de la séquence de formule :XGM-D-E--VL-W-F-S-LA--H-ARE-HPE--K-CZ
dans laquelle X et Z sont des groupements OH ou $NH_2$ ou, dans la mesure où les propriétés immunologiques du peptide dépourvu de ces groupes ne s'en trouvent pas essentiellement modifiées, des groupes comportant de 1 à 5 résidus d'acides aminés, et chacun des tirets correspond à un résidu aminoacyle choisi parmi ceux qui permettent de conserver au peptide sus-défini la capacité d'être reconnu par des sérums humains contenant des anticorps contre la protéine F d'un rétrovirus HIV-1 et plus particulièrement des anticorps monospécifiques dirigés contre le peptide ci-dessus.

3. Peptide selon la revendication 1 ou la revendication 2, caractérisé en ce qu'il est constitué par tout ou partie d'un peptide choisi parmi les peptides suivants :
GMDDPEREVLEWRFDSRLAFHHVARELHPEYFKNC
GMEDAEKEVLVWRFDSKLAFHHMARELHPEYYKDC
GMEDAEREVLKWKFDSSLALRHRAREQHPEYYKDC
GMEDPERQVLKWRFNSRLAFEHKAREMHPEFYKN

4. Peptide selon la revendication 1, caractérisé en ce qu'il est choisi parmi les suivants :
PFII : XGGKWSKSSVVGWPTVRERMRRAEPAADGVGAYCZ
PF12 : XGGKWSKSSVVGWPTVRERMRRAEPAADGVGAASRDLEKHGAITSSNTAATNAAC AWLEA-QEEEEVG
PF13 : XASRDLEKHGAITSSNTAATNAACAWLEAQEEEEZ
PF14 : XCVGFPVTPQVPLRPMTYKAAVDLSHFLKEKGGLEGLIHSQRRQDILZ
PF15 : XCGYFPDWQNYTPGPGVRYPLTFGWCYKLVPVEPDKVEEANKGENTSLLHPVZ
PF16 : XGMDDPEREVLEWRFDSRLAFHHVARELHPEYFKNCZ
PF17 : XCYKLVPVEPDKVEEANKGENTSLLHPVSLHGMDDPEREVLEWRFDSRLAFHHVAR ELHPEYFKNCZ
PF18 : XCKGGLEGLIHSQRRQDILDLWIYHTQGYFPDZ
dans lesquels X et Z sont des groupements OH ou $NH_2$ ou dans la mesure où les propriétés immunologiques du peptide dépourvu de ces groupes ne s'en trouvent pas essentiellement modifiées, des groupes comportant de 1 à 5 résidus d'acides aminés, choisis de telle sorte qu'ils permettent de conserver au peptide sus-défini la capacité d'être reconnu par des sérums humains contenant des anticorps contre la protéine F d'un rétrovirus HIV-1 et plus particulièrement des anticorps monospécifiques dirigés contre les peptides ci-dessus.

5. Peptide selon la revendication 1, caractérisé en ce qu'il présente des propriétés immunologiques, en commun avec celles de la séquence suivante :
PF10 : XKFDDPHGETLVWEFDPLLAYSYEAFIRYPEEFGHKZ
dans laquelle X et Z sont des groupements OH ou $NH_2$ ou, dans la mesure où les propriétés immunologiques du peptide dépourvu de ces groupes ne s'en trouvent pas essentiellement modifiées, des groupes comportant de 1 à 5 résidus d'acides aminés, choisis de telle sorte qu'ils permettent de conserver au peptide sus-défini la capacité d'être reconnu par des sérums humains contenant des anticorps contre la protéine F d'un rétrovirus HIV-2 et plus particulièrement des anticorps monospécifiques dirigés contre le peptide ci-dessus.

6. Peptide selon la revendication 1, caractérisé en ce qu'il présente des propriétés immunologiques en commun avec celles de la séquence suivante :
PF19 : XCRGGLEGMFYSERRHKILNIYLEKEEGIIADZ
dans laquelle X et Z sont des groupements OH ou $NH_2$ ou, dans la mesure où les propriétés immunologiques du peptide dépourvu de ces groupes ne s'en trouvent pas essentiellement modifiées, des groupes comportant de 1 à 5 résidus d'acides aminés, choisis de telle sorte qu'ils permettent de conserver au peptide sus-défini la capacitité d'être reconnu par des sérums humains contenant des anticorps contre la protéine F d'un rétrovirus HIV-2 et plus particulièrement des anticorps monospécifiques dirigés contre le peptide ci-dessus.

7. Composition pour la détection d'anticorps dirigés contre la protéine F ou contre une partie de la protéine F produite lors d'une infection par un rétrovirus HIV-1, caractérisée en ce qu'elle comprend en mélange, au moins deux peptides différents choisis parmi les peptides selon l'une quelconque des revendications 1 à 4.

8. Composition pour la détection d'anticorps dirigés contre la protéine F ou contre une partie de la protéine F produite lors d'une infection par un rétrovirus HIV-2, caractérisée en ce qu'elle comprend en

mélange, au moins deux peptides différents choisis parmi les peptides selon l'une quelconque des revendications 5 ou 6.

9. Composition pour la détection d'anticorps dirigés contre la protéine F ou contre une partie de la protéine F produite lors d'une infection par un rétrovirus HIV, caractérisée en ce qu'elle comprend en mélange, au moins deux peptides différents choisis parmi les peptides selon les revendications 1 à 6.

10. Composition selon la revendication 7 ou la revendication 8, caractérisée en ce qu'elle comprend en mélange au moins deux peptides choisis pour l'un d'entre eux parmi les peptides de la région C-terminale de la protéine F de HIV-1, par exemple un peptide choisi parmi les séquences de PF15, PF16 et PF17 et pour l'autre, parmi les peptides de la région N-terminale de la protéine de HIV-1, par exemple un peptide choisi parmi les séquences de PF11, PF12 et PF13.

11. Composition selon la revendication 10, caractérisée en ce qu'elle comprend en mélange les peptides PF12 et PF17.

12. Composition selon la revendication 11, caractérisée en ce qu'elle comprend en mélange les peptides PF12 et PF16.

13. Composition selon la revendication 11, caractérisée en ce qu'elle comprend en mélange les peptides PF13 et PF17.

14. Composition selon la revendicatioçn 11, caractérisée en ce qu'elle comprend en mélange les peptides PF13 et PF16.

15. Composition de peptides caractérisée en ce qu'elle comprend un mélange d' au moins un peptide selon l'une quelconque des revendications 2 à 4 et d'un peptide selon la revendication 5 ou la revendication 6.

16. Kit pour le diagnostic in vitro d'une infection par un rétrovirus capable de provoquer un SLA ou un SIDA, caractérisé en ce qu'il comprend :

- un peptide selon l'une quelconque des revendications 1 à 6, ou une composition de peptides selon l'une quelconque des revendications 7 à 15 ;
- les réactifs pour la constitution d'un milieu, notamment une solution tamponnée, propice à la formation d'une réaction immunologique entre le ou les peptides et les anticorps éventuellement présents dans un échantillon biologique ;
- un ou plusieurs réactifs éventuellement marqué(s) aptes(s) à réagir avec le ou lesdits peptides pour la détection du complexe peptideanticorps formé ;
- le cas échéant, un milieu biologique de référence, tel qu'un sérum, semblable au milieu biologique provenant de la personne éventuellement infectée avec un rétrovirus HIV et dépourvu d'anticorps reconnaissant les susdits peptides.

17. Anticorps monospécifiques dirigés contre la protéine F, caractérisés en ce qu'ils sont reconnus immunologiquement par un ou plusieurs peptides selon l'une des revendications 1 à 6 ou par des compositions selon l'une quelconque des revendications 7 à 15.

18. Procédé de synthèse d'un peptide selon l'une quelconque des revendications 1 à 6, caractérisé en ce qu'il comprend par les étapes suivantes :

a/ mise en contact d'un support solide portant un groupement réactif apte à réagir avec une fonction carboxyle, avec le premier acide aminé C-terminal de la chaîne à synthétiser, protégé au niveau de la fonction amine devant ultérieurement intervenir dans le couplage avec un autre acide aminé, pour éviter l'auto-condensation ;

b/ déprotection de la fonction amine de l'acide aminé précédemment fixé ;

c/ couplage de la fonction amine déprotégée de l'acide aminé à l'issue de l'étape b/ avec la fonction carboxyle d'un deuxième acide aminé, protégé au niveau de sa fonction amine et qui fournit le deuxième résidu aminoacyle alors couplé au résidu aminoacyle C-terminal du peptide à synthétiser ;

d/ déprotection de la fonction amine du deuxième acide aminé ajouté et répétition des étapes c/ de couplage et d/ de protection, successivement avec les acides aminés correspondant respectivement aux résidus aminoacyle successifs jusqu'à aboutir au résidu aminoacycyle N-terminal dudit peptide ;

e/ séparation du peptide formé, par traitement de la phase solide portant le peptide synthétisé, notamment par exemple au moyen de l'acide fluorhydrique, après incorporation de l'acide-aminé N-terminal ;

f/ récupération de la séquence peptidique à partir de la solution.

19. Procédé de synthèse selon la revendication 18, caractérisé en ce que l'étape b/ de déprotection est suivie par une étape de neutralisation destinée à rendre la fonction amine nucléophile.

20. Procédé de synthèse selon la revendication 18 ou 19, caractérisé en ce que l'étape de couplage fait intervenir un agent de couplage capable d'activer la fonction carboxyle, de préférence le dicyclohexylcarbodiimide.

21. Procédé de synthèse selon l'une quelconque des revendications 18 à 20, caractérisé par une seconde étape de couplage réalisée après lavage et neutralisation du peptide obtenu après l'étape c/, au moyen d'un agent de couplage, afin de permettre la réaction des fonctions amines qui n'avaient pas réagi lors du premier couplage.

22. Procédé de synthèse selon l'une quelconque des revendications 18 à 21, caractérisé en ce que la

protection des fonctions tertiaires des acides aminés est réalisée par le 2,6-dichlorobenzyle pour la fonction OH de la tyrosine, le benzyle pour la fonction OH de la thréonine ou de la sérine ou pour la fonction COOH de l'aspartate ou du glutamate, le t-butylmercapto pour la fonction SH de la cystéine, le tosyle pour l'imidazole de l'histidine ou la fonction NH de l'arginine, le benzyloxycarbonyle pour la fonction $NH_2$ de la lysine, le formyle pour la fonction indole du tryptophane;

23. Procédé de détection in vitro d'une infection par un rétrovirus HIV susceptible de provoquer un SLA ou un SIDA, caractérisé en ce qu'il comprend :

a/ la mise en contact d'au moins un peptide selon l'une quelconque des revendications 1 à 6 ou d'une composition selon l'une quelconque des revendications 7 à 15 avec un échantillon biologique à tester, susceptible de contenir des anticorps contre la protéine F d'un rétrovirus HIV, dans des conditions permettant la formation d'un complexe immunologique peptide-anticorps,

b/ la détection du complexe immunologique formé.

24. Procédé de détection selon la revendication 22, caractérisé en ce que le peptide mis en oeuvre reconnaît spécifiquement des anticorps formés contre la protéine F d'un rétrovirus HIV-1.

25. Procédé de détection selon la revendication 23, caractérisé en ce que le peptide mis en oeuvre reconnaît spécifiquement des anticorps formés contre la protéine F d'un rétrovirus HIV-2.

26. Procédé selon la revendication 23, caractérisé en ce que l'on met en oeuvre un mélange de peptides, capable de détecter une infection par un rétrovirus HIV-1 ou HIV-2.

PRTALN

alignement de :

FRODP

VERSUS

F.LAV.P

```
+++++++++++++++++++++++++++++++++++++++++++++++++++

         10        20        30                  40    50
MGASGSKKHSRPPRGLQERLLRARAGACGGYWNESG--G-----EYSRFQEGSDREQKSP    HIV.2
                              :: :  :   :     : : :
                              MGGKWSKSSVVGWPTVRERMRRAEPAADGVGAA  HIV.1
                                        10        20        30

      60        70                 80        90       100
SCEGRQYQQGDFMNTPWKDPAA-------EREKNLYRQQNMDDVDSDDDDQVRVSVTPKV
:               ::    :         : :                    :  ::: :
SRDLEKHGAITSSNTAATNAACAWLEAQEE-EE------------------VGFPVTPQV
      40        50        60                            70

   110       120       130       140       150       160
PLRPMTHRLAIDMSHLIKTRGGLEGMFYSERRHKILNIYLEKEEGIIADWQNYTHGPGVR
::::::   : : ::  :  :::::   : ::  ::        :   :::::: :::::
PLRPMTYKAAVDLSHFLKEKGGLEGLIHSQRRQDILDLWIYHTQGYFPDWQNYTPGPGVR
   80        90       100       110       120       130

   170       180       190       200       210       220
YPMFFGWLWKLVPV--DVPQEGEDTETHCLVHPAQTSKFDDPHGETLVHEFDPLLAYSYE    HIV.2
::  :::  :::::  :   :   :     : ::   ::: : : : :: ::
YPLTFGWCYKLVPVEPDKVEEANKGENTSLLHPVSLHGMDDPEREVLEWRFDSRLAFHHV    HIV.1
   140       150       160       170       180       190

     230        240       250
AFIRYPEEFGHKSGLPEEEWKARLKARGIPFS
:    :: :
ARELHPEYFKNC
    200
```

nombre d'acides aminés semblables : 82

**FIGURE 1**

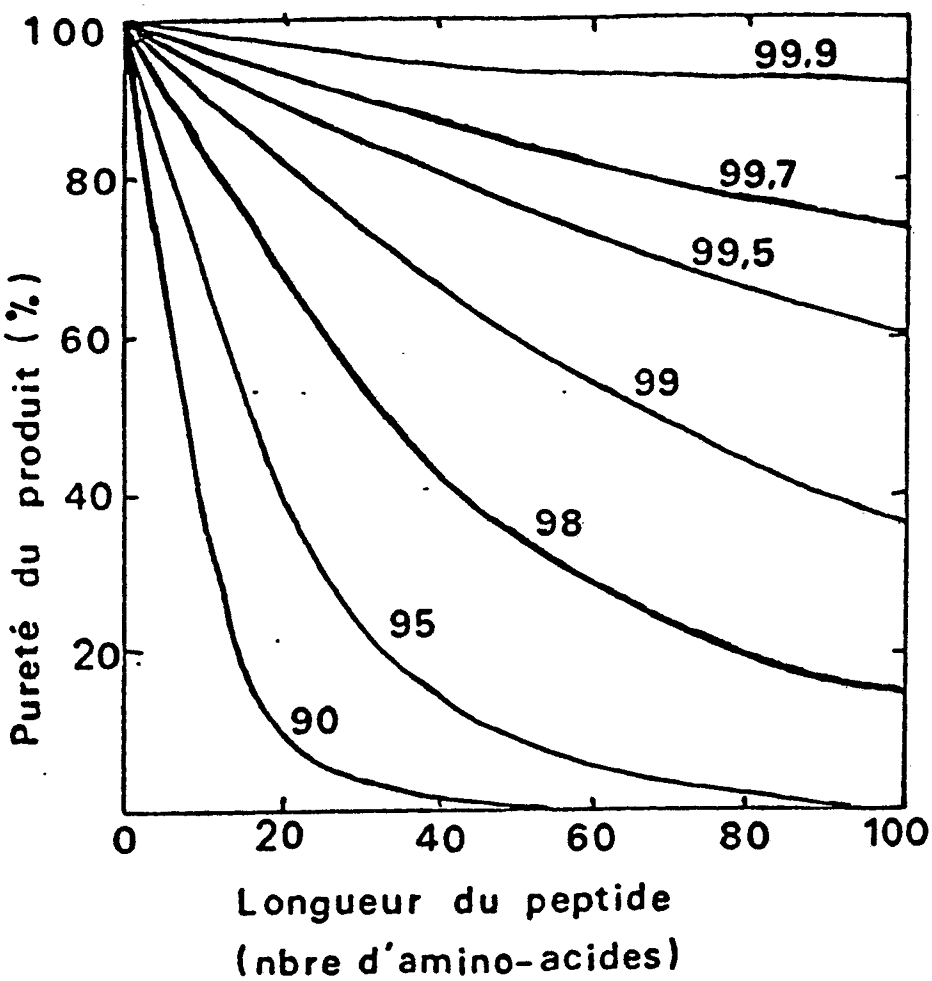
100
80
60
40
20
Pureté du produit (%)
99,9
99,7
99,5
99
98
95
90
0
20
40
60
80
100
Longueur du peptide
(nbre d'amino-acides)

Figure 2

F

```
                  10         20         30         40         50         60         70         80
LAV BRU   MGGKWSKSSV VGWPTVRERM R----RAEPA ADGVGAASR- ----DLEKHG AITSSNTAAT NAACAWLEAQ EE-EEVGFPV
ARV 2            R N G  SAI      RAEP            V  - ----                    D          -
LAV MAL            I     KI   I  ---- TP T ET    V QD AVSQ  D C   AA  SP  N   S --- PP   E
LAV ELI            I     AI   I  ---- TN         V  - ----               S    D           SD

                  90        100        110        120        130        140        150        160
LAV BRU   TPQVPLRPHT YKAAVDLSHF LKEKGGLEGL IHSQRRQDIL DLWIYHTQGY FPDWQNYTPG PGVRYPLTFG WCYKLVPVEP
ARV 2     R              L I                W     E                           I          F
LAV MAL   R            G F             D   VW PK  E       V                   I F        F    NS
LAV ELI   R            E L                  W KK  E       V N  I              I           E    D

                 170        180        190        200        210
LAV BRU   DKVEEANKGE NTSLLHPVSL HGMDDPEREV LEWRFDSRLA FHHVARELHP EYFKNC
ARV 2     E      E    N     H      E A K    V     K      M         Y D
LAV MAL   EE     E    NC     I Q   E A      K K   S   LR R   Q     Y D
LAV ELI   QE   DTE   TN     ICQ    E    Q   K   N      E K   M    FY  -
```

FIGURE 3

HIV-1

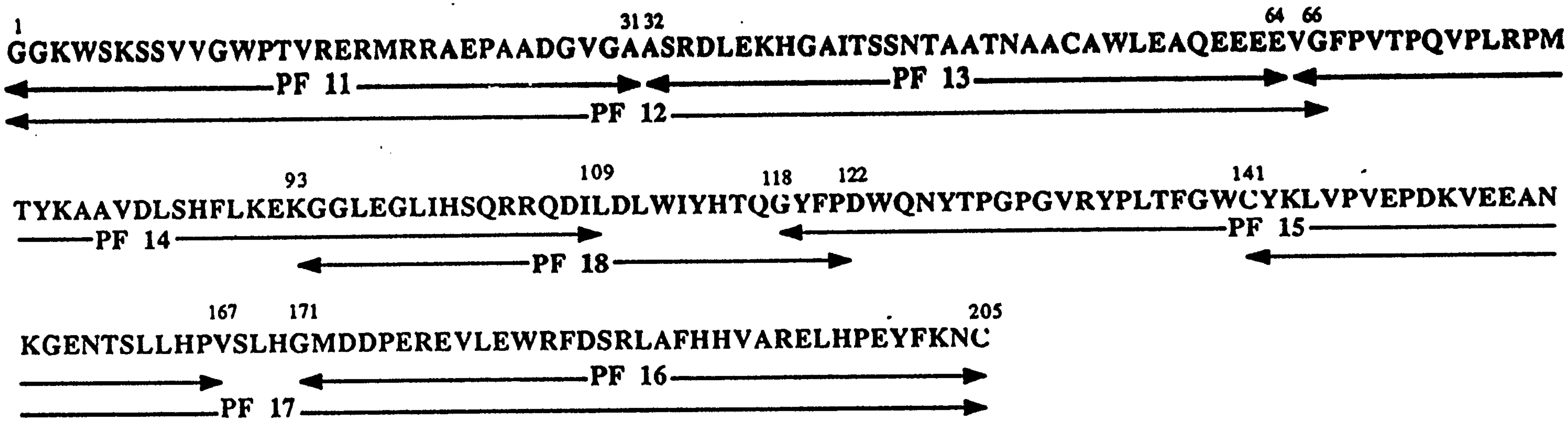


Figure 4

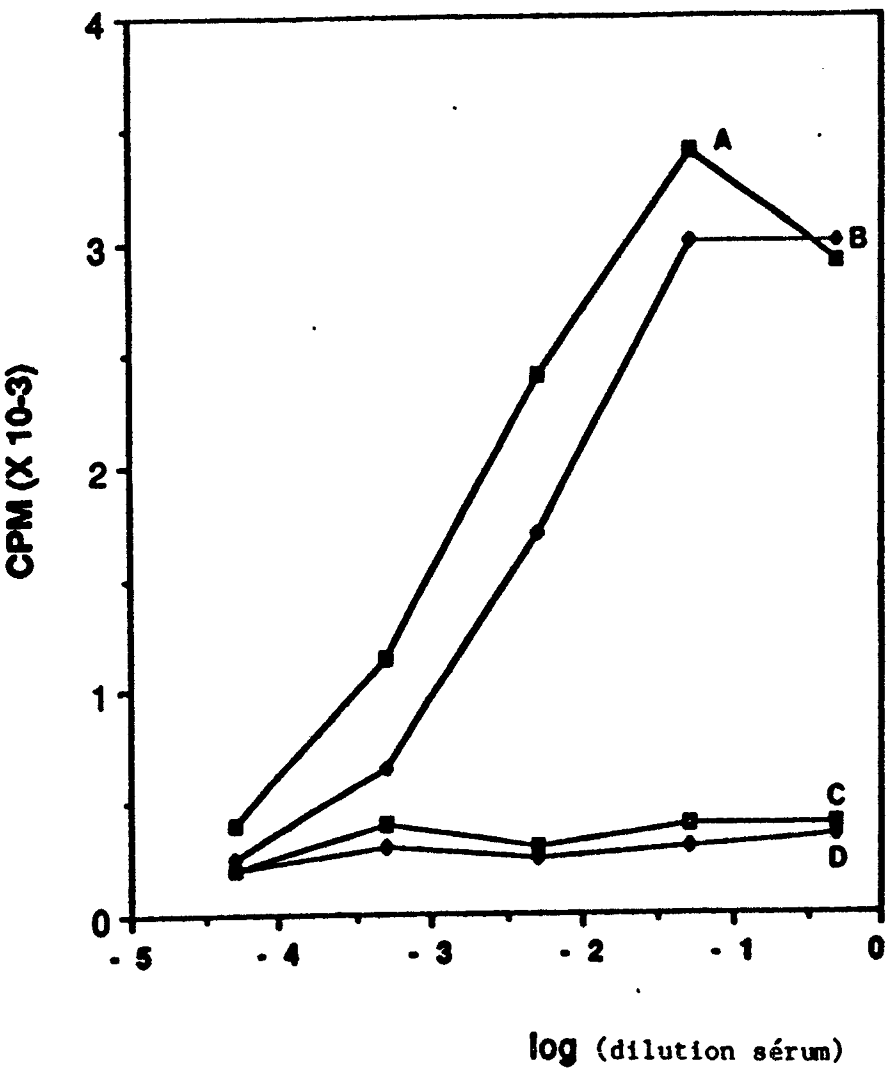


Figure 5

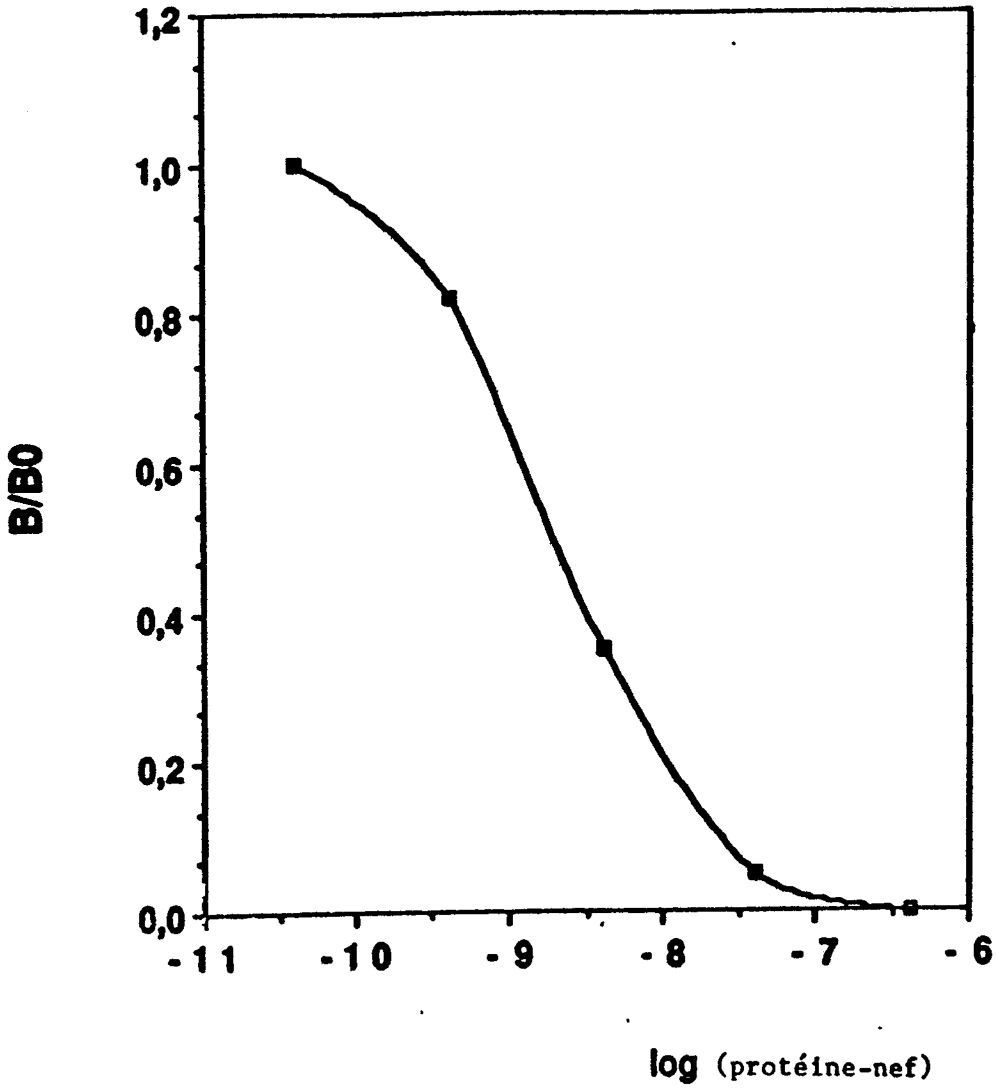


Figure 6

Office européen des brevets

# RAPPORT DE RECHERCHE EUROPEENNE

Numero de la demande

EP 89 40 0905

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | WO-A-8 707 642 (TRANSGENE S.A.)<br>* En entier *<br>--- | 1-17,23-26 | C 07 K 7/00<br>G 01 N 33/569<br>C 07 K 1/00<br>C 07 K 15/00 |
| A | EP-A-0 242 216 (THE UNITED STATES OF AMERICA)<br>* Résumé; revendications *<br>--- | 1-17,23-26 | |
| A | C.R. ACAD. SC. PARIS, vol. 302, série III, no. 8, 1986, pages 287-292, Académie des Sciences, Paris, FR; C. AUFFRAY: "Immunologie - Un modfle moléculaire de l'interaction entre l/antigène T4 et les antigènes HLA de la classe II ou le virus LAV"<br>* En entier *<br>--- | 1-17,23-26 | |
| A | PROC. NATL. ACAD. SCI., USA, vol. 83, avril 1986, pages 2209-2213; S.K. ARYA et al.: "Three novel genes of human T-lymphotropic virus type III: immune reactivity of their products with sera from acquired immune deficiency syndrome patients"<br>* Résumé *<br>--- | 1-17,23-26 | **DOMAINES TECHNIQUES RECHERCHES (Int. Cl.4)** |
| A | NATURE, vol. 330, 19 novembre 1987, pages 266-269; B. GUY et al.: "HIV F/3' orf encodes a phosphorylated GTP-binding protein resembling an oncogene product"<br>* Résumé; figure 1 *<br>--- -/- | 1-17,23-26 | A 61 K<br>C 12 N<br>G 01 N<br>C 07 K |

Le présent rapport a été établi pour toutes les revendications

| Lieu de la recherche | Date d'achèvement de la recherche | Examinateur |
|---|---|---|
| LA HAYE | 08-08-1989 | OSBORNE H.H. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

Office européen des brevets

# RAPPORT DE RECHERCHE EUROPEENNE

Numero de la demande

EP 89 40 0905

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | FEBS LETTERS, vol. 218, no. 1, juin 1987, pages 81-86, Elsevier Science Publsishers B.V. (Biomedical Division), Amsterdam, NL; K.P. SAMUEL et al.: "The 3'-orf protein of human immunodificiency virus shows structural homology with the phosphorylation domain of human interleukin-2 receptor and the ATP-binding site of the protein kinase family"<br>* Résumé; figure 1 *<br>--- | 1-17,23<br>-26 | |
| A | EP-A-0 187 041 (GENENTECH INC.)<br>* Résumé; revendications 1,8-10,47-63 *<br>--- | 1-17,23<br>-26 | |
| A | EP-A-0 226 513 (INSTITUT PASTEUR)<br>* En entier *<br>--- | 1-17,23<br>-26 | |
| A | SCIENCE, vol. 230, 15 november 1985, pages 810-813; J.S. ALLAN et al.: "A new HTLV-III/LAV encoded antigen detected by antibodies from AIDS patients"<br>* En entier *<br>--- | 1-17,23<br>-26 | DOMAINES TECHNIQUES RECHERCHES (Int. Cl.4) |
| A | FEBS LETTERS, vol. 222, no. 2, 5 octobre 1987, pages 286-288, Elsevier Science Publishers B.V. (Biomedical Division), Amsterdam, NL; B. MAURER et al.: "The 3'-orf protein of human immunodeficiency virus 2 shows sequence homology with the bel3 gene of the human spumaretrovirus"<br>* En entier *<br>----- | 1-17,23<br>-26 | |

Le présent rapport a été établi pour toutes les revendications

| Lieu de la recherche | Date d'achèvement de la recherche | Examinateur |
|---|---|---|
| LA HAYE | 08-08-1989 | OSBORNE H.H. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)